# EUROPEAN PATENT SPECIFICATION

(11) **EP 4 469 518 B1**
(45) Date of publication and mention of the grant of the patent: **10.12.2025**
(21) Application number: 23711818.7
(22) Date of filing: 25.01.2023
(51) Int. Cl.: C08L 67/04, A61K 35/644

(54) **BIOCOMPOSITE COMPRISING BEESWAX AND METHOD OF OBTAINING BIOCOMPOSITE USING BEESWAX**
BIOVERBUNDSTOFF MIT BIENENWACHS UND VERFAHREN ZUR HERSTELLUNG EINES BIOVERBUNDSTOFFS MIT BIENENWACHS
BIOCOMPOSITE COMPRENANT DE LA CIRE D'ABEILLE ET PROCÉDÉ D'OBTENTION DE BIOCOMPOSITE À L'AIDE DE CIRE D'ABEILLE

(30) Priority: 27.01.2022 PL 44024122
(43) Date of publication of application: 04.12.2024
(73) Proprietor: POLITECHNIKA WARSZAWSKA, 00-661 Warszawa (PL); Uniwersytet Im. Adama Mickiewicza W Poznaniu, 61-712 Poznan (PL)
(72) Inventor: PRZEKOP, Robert, 62-002 Suchy Las (PL); DOBRUCKA, Renata, 60-218 Poznan (PL); DOBROSIELSKA, Marta, 61-619 Poznan (PL); KURZYDLOWSKI, Krzysztof, 18-100 Lapy (PL)
(74) Representative: Kawczynska, Marta Joanna
(86) International application number: PCT/PL2023/050004
(87) International publication number: WO 2023/146424

(56) References cited:
- CN-A- 106 519 619
- DOBROSIELSKA MARTA ET AL: "Biogenic Composite Filaments Based on Polylactide and Diatomaceous Earth for 3D Printing", MATERIALS, vol. 13, no. 20, 16 October 2020 (2020-10-16), pages 4632, XP093047903, DOI: 10.3390/ma13204632
- AGUERO ANGEL ET AL: "Effect of different compatibilizers on environmentally friendly composites from poly(lactic acid) and diatomaceous earth", POLYMER INTERNATIONAL, vol. 68, no. 5, 28 February 2019 (2019-02-28), GB, pages 893 - 903, XP093047906, ISSN: 0959-8103, Retrieved from the Internet <URL:https://onlinelibrary.wiley.com/doi/full-xml/10.1002/pi.5779> DOI: 10.1002/pi.5779
- DOBROSIELSKA MARTA ET AL: "Beeswax as a natural alternative to synthetic waxes for fabrication of PLA/diatomaceous earth composites", SCIENTIFIC REPORTS, vol. 13, no. 1, 20 January 2023 (2023-01-20), XP093047902, Retrieved from the Internet <URL:https://www.nature.com/articles/s41598-023-28435-0> DOI: 10.1038/s41598-023-28435-0

## Description

The invention relates to a biocomposite consisting of biodegradable poly(lactic acid), diatomaceous earth and beeswax, and a method of obtaining the composite using beeswax. The use of beeswax for the production of the biocomposite is also disclosed. The use of beeswax has made it possible to eliminate synthetic (petroleum-derived) waxes used as a processing additive.

Composite materials are materials that have a heterogeneous structure and consist of at least two phases. Their combination results in a material that has different properties than the properties of the individual initial components. Most often, a composite material consists of two components, i.e. a structural element and a filling element, but sometimes additional components can also be used. The structural component, i.e. the main component constituting the continuous phase consolidates fragments of the extender, enables the products to be appropriately shaped and determines most of the physical and chemical properties of the material. The function of the disperse phase is to improve selected properties of the material. An additional component is used to enhance interaction between the matrix and the extender and thereby it affects the cohesion and homogeneity of the material. Typical composites are based on polymers such as styrene polymers, polycarbonate, thermoplastic polyesters, polypropylene, the production of which is not ecological.

In the last few decades, conventional polymer composites for various applications have been more and more often replaced with composites obtained with the use of renewable raw materials called biocomposites.

Due to the necessity of limiting the production of plastic waste and emphasis on sustainable production methods and reduced energy consumption, the trend to develop environmentally friendly polymers and polymer composites has been dominating in recent years. Composite materials are materials that comprise one or more phases of organic origin. The market for the production and sale of composites, which has been observed for years, contributes to the intensification of research work related to their production and modification. That is why biopolymers obtained from natural resources currently represent an alternative to petroleum-derived polymers. They may also contribute to the reduction of the carbon footprint of a product. Among biopolyesters, polylactide (PLA) has a well-established position due to its processability, sustainable properties and a relatively low price.

The publication of Aguero A et al., 2019 presents environmentally friendly composites of poly(lactic acid) (PLA) and diatomaceous earth (DE) produced by the method of extrusion and then injection moulding. DE was used as filler. Several agents playing the role of compatibilizers / coupling agents, such as (3-glycidyloxypropyl)trimethoxysilane, epoxy styrene-acrylic oligomer and maleinized linseed oil were used in order to improve the polymer-filler interaction. DE ensures a better elasticity module during stretching and results in more brittle composites due to the phenomenon of stress concentration. A compatibilizer on the other hand contributes to the improvement in ductile properties resulting in materials having a high environmental performance, with sustainable mechanical properties. The publication of Dobrosielska et al., 2020 presents new composites of polylactide (PLA) comprising a natural filler made of shell (cell wall) of diatoms (frustules) and enabling modification of a matrix composed of PLA, produced by the method of Fused Deposition Modelling (3D print), and describes a method for preparing them.

The application WO2012106006A1 discloses a biolaminate composite assembly comprising one or more layers of biolaminate, wherein at least one layer comprises poly(lactic acid) and natural wax, wherein the wax is soy wax which constitutes about 5% by weight of a biolaminate composite assembly.

The publication of Righetti et al., 2019 describes a biocomposite produced from a polymeric matrix comprising 85% by weight of PLA, 10% by weight of plasticizer, acetyl tri-n-butyl citrate (ATBC) and CaCO₃ having 5% concentration by weight, to which subsequently 20% by weight of potato pulp powder was added. In order to obtain the biocomposite based on PLA with potato pulp powder and covered with wax, commercially available non-ionic aqueous emulsion of beeswax, carnauba wax or modified polypropylene wax in the concentration of 5% w/v was added to the pulp.

CN 106 519 619 A discloses compositions comprising PLA, DE, and beeswax.

In connection with the search for an alternative to conventional polymer composites, in this study a composite material consisting solely of biodegradable poly(lactic acid), diatomaceous earth and beeswax was obtained. The invention developed is a three-component material consisting only of natural raw materials: a biopolymer which is PLA, a naturally occurring extender (diatomaceous earth) and beeswax.

The objective of the present invention is the use of natural beeswax as an agent improving the processing properties (melt flow rate) for the production processes of composite materials based on PLA and diatomaceous earth, and thus improvement of the state-of-the art composite materials. The use of beeswax in the described composite system provides it with better rheological properties compared to polyamide waxes commonly used the processing industry, and at the same time it is less allergenic and of fully natural origin. In the invention, the wax is an independent rheological factor modifying viscosity and ensuring a uniform structure of the biocomposite in a two-component system with PLA.

The composite material obtained with the use of beeswax, produced using standard processing technologies, has improved mechanical properties and processing capabilities, which offers a wide spectrum of its application in many branches of industry and is a response to the currently existing trends related to the search of new solutions for traditional plastics. Due to its bio-origin, the wax used does not have a carbon footprint, is non-toxic, physiologically neutral and thus can be, unlike synthetic processing additives, used for products that come into contact with food, human skin, for the production of animal feeding dishes, etc., toys, household items, etc. Wax as an additive in the processing industry of plastics (biopolymer polylactide, PLA) increases MFR - mass flow rate, and thus increases the processing rate in the processes of injection, extrusion, blow moulding, pressing or pouring.

The invention is defined by the appended claims.

The invention relates to a biocomposite of biodegradable poly(lactic acid) and diatomaceous earth, characterized in that it additionally comprises beeswax, wherein the content of diatomaceous earth is 2.5-15% of the mixture and the content of beeswax is 0.5 - 1% of the mixture.

The invention also relates to a method of producing the biocomposite, wherein poly(lactic acid) in the form of granulate, crude diatomaceous earth and beeswax are homogenized in the temperature range from 180 0°C to 230°C, preferably 205°C - 215°C, subsequently the components are milled, the product of milling is injected at a temperature of 195°C - 210°C, subsequently the granulates are diluted 1:1 with pure polylactide and a composite is obtained which is conditioned for 5 days at room temperature and/or 5 days in a climatic chamber in 10 heating-cooling cycles (+50 °C, -10 °C).

In accordance with the invention, the use of beeswax for obtaining the biocomposite has been described wherein the wax is an independent rheological factor in the two-component system with poly(lactic acid).

### Description of the Figures

Fig. 1 shows the results for the melt flow rate of the granulates after the injection process.
Fig. 2 shows the tensile strength of the composites conditioned at room temperature.
Fig. 3 shows elongation at break of the composites conditioned at room temperature.
Fig. 4 shows the results of tests for bending strength (elasticity module) for the composites conditioned at room temperature.
Fig. 5. shows the results of tests for bending strength (maximum bending stress) for the composites conditioned at room temperature.
Fig. 6 shows the impact strength of the composites conditioned at room temperature.
Fig. 7 shows the tensile strength of the composites conditioned in the climatic chamber for 5 days.
Fig. 8. shows the elongation at break of the composites conditioned for 5 days in the climatic chamber.

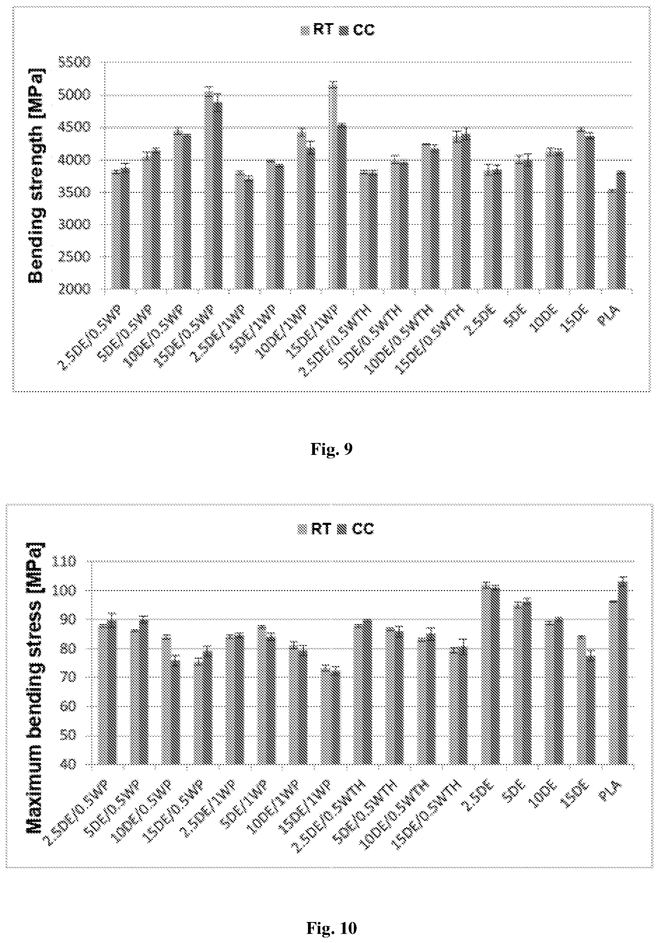

Fig. 9 shows the results of tests for bending strength (elasticity module) for the composites conditioned for 5 days in the climatic chamber.
Fig. 10. shows the results of tests for bending strength (maximum bending stress) for the composites conditioned for 5 days in the climatic chamber.

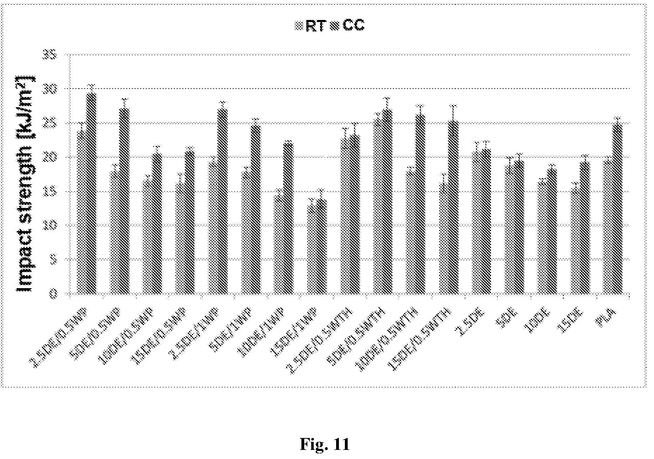

Fig. 11. shows the impact strength of the composites conditioned in the climatic chamber for 5 days.

### Examples

### Example 1

### Obtaining the biocomposite

The composite was obtained by the homogenization of components in the form of PLA4043D granulate (Minnetonka, MN, USA), crude diatomaceous earth (Perma Guard, Inc., Bountiful, UT 4010, USA) and beeswax at a temperature from 180°C to 230°C, preferably at a temperature of 205°C to 215°C.

The wax used contains 100% natural beeswax, is a product melted from a 'honeycomb'. After extracting honey, the comb [the frame with dry honeycomb] is heated in order to melt beeswax. Beeswax has a relatively law melting temperature, which is from 62°C to 64°C. The material used for the test originated from the APIS Apiculture Cooperative in Lublin. It was wax (in the form of 5 kg blocks) standardized at the temperature of 210°C with the use of a laboratory rolling mill (VM-150/280 Zamak Mercator).

Mixtures with a variable content of diatomaceous earth (5%, 10%, 20%, 30%) and 1% and 2% of beeswax or synthetic WTH-B wax or without the addition of wax were obtained. Subsequently, the obtained systems were milled by means of a mill for plastics. Samples for strength tests were prepared by means of an injection moulder at a temperature of 195°C - 210°C by diluting the prepared mixtures 1:1 with pure PLA4043D and obtaining systems with 2.5%, 5%, 10% and 15% content of diatomaceous earth and 0.5% or 1% content of beeswax. The same method was also used to prepare systems containing 0.5% of synthetic WTH-B wax and systems unmodified with an addition of waxes.

| Name of system | Abbreviated name | % DE content | % beeswax content | % synthetic wax (WTH-B) content |
|---|---|---|---|---|
| PLA4043D | PLA | 0 | 0 | 0 |
| PLA4043D + 2.5% DE + 0.5% WP | 2.5DE/0.5WP | 2.5 | 0.5 | 0 |
| PLA4043D + 5% DE + 0.5% WP | 5DE/0.5WP | 5 | 0.5 | 0 |
| PLA4043D + 10% DE + 0.5% WP | 10DE/0.5WP | 10 | 0.5 | 0 |
| PLA4043D + 15% DE + 0.5% WP | 15DE/0.5WP | 15 | 0.5 | 0 |
| PLA4043D + 2.5% DE + 1% WP | 2.5DE/1WP | 2.5 | 1 | 0 |
| PLA4043D + 5% DE + 1% WP | 5DE/1WP | 5 | 1 | 0 |
| PLA4043D + 10% DE + 1% WP | 10DE/1WP | 10 | 1 | 0 |
| PLA4043D + 15% DE + 1% WP | 15DE/1WP | 15 | 1 | 0 |
| PLA4043D + 2.5% DE + 0.5% WTH | 2.5DE/0.5WTH | 2.5 | 0 | 0.5 |
| PLA4043D + 5% DE + 0.5% WTH | 5DE/0.5WTH | 5 | 0 | 0.5 |
| PLA4043D + 10% DE + 0.5% WTH | 10DE/0.5WTH | 10 | 0 | 0.5 |
| PLA4043D + 15% DE + 0.5% WTH | 15DE/0.5WTH | 15 | 0 | 0.5 |
| PLA4043D + 2.5% DE | 2.5DE | 2.5 | 0 | 0 |
| PLA4043D + 5% DE | 5DE | 5 | 0 | 0 |
| PLA4043D + 10% DE | 10DE | 10 | 0 | 0 |
| PLA4043D + 15% DE | 15DE | 15 | 0 | 0 |

| | | | | |
|---|---|---|---|---|
| DE - diatomaceous earth WP - beeswax WTH - synthetic WTH-B wax | | | | |

### Example 2

Testing of the melt flow rate (MFR. 210°C, load 2.16 kg) for the produced biocomposites. Composites for the strength tests obtained by injection were milled in a mill for plastics and were dried at the temperature of 60°C for 24 h before testing.

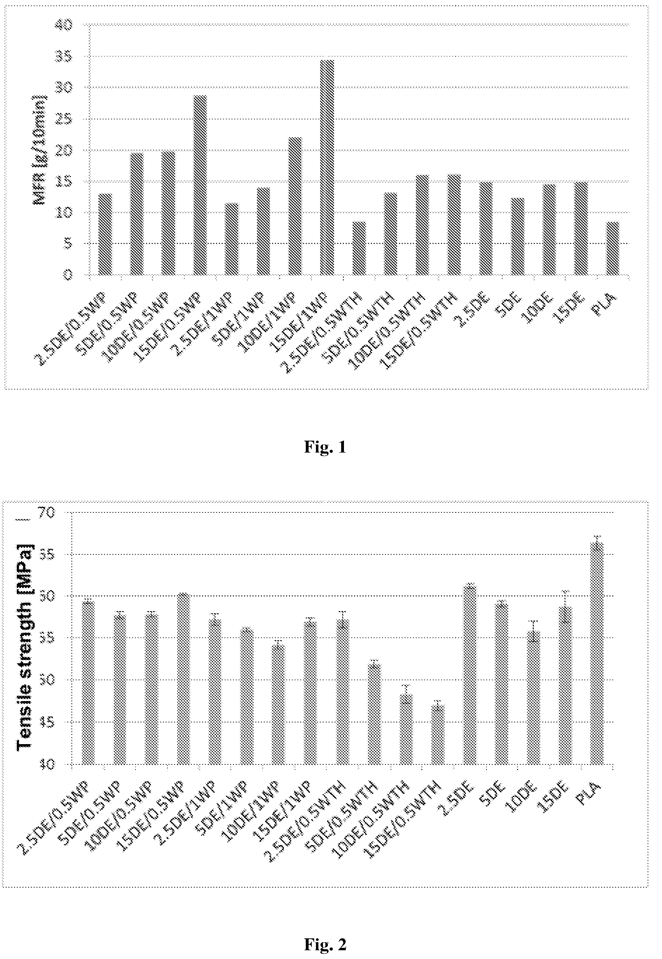

The melt flow rate (MFR) determined for unmodified PLA4043D was 8.47 g/10 min. The addition of diatomaceous earth (DE) only, irrespective of the concentration, caused an increase of the MFR parameter, at the concentrations of 10% and 15% - almost double increase. The addition of synthetic WTH-B wax Microbeads also caused an increase of MFR, but up to the maximum value of 14.9 g/10 min for the highest concentration of diatomaceous earth. The increase was analogous to that for the systems without the addition of wax, so an additional influence of the synthetic wax on the rheology of the composite in the melted (fluid) state was not observed; on the contrary, at higher concentrations, higher MFR values are observed for the composites without the addition of wax. The addition of natural wax, irrespective of the concentration (0.5%, 1%), each time caused a linear increase of the melt flow rate up to the value of 34.4 g/10 min (over 4-fold increase) for 1% concentration of the additive (Fig. 1, Tab. 1.).

**Table 1. Melt flow rate (MFR) for granulates after injection process.**

| **MFR 210°C [g/10min]** | **0.5 % WP** | **1% WP** | **Without wax** | **0.5 % WTH** |
|---|---|---|---|---|
| **2.5% DE** | 12.990 | 11.461 | 8.501 | 14.863 |
| **5% DE** | 19.527 | 13.997 | 13.103 | 12.304 |
| **10% DE** | 19.755 | 22.054 | 16.077 | 14.528 |
| **15% DE** | 28.728 | 34.446 | 16.137 | 14.889 |
| **PLA** | 8.477 | | | |

### Example 3

Mechanical tests (breaking strength, bending strength, impact strength) of the obtained biocomposites.

Biocomposites produced by the injection method were conditioned at room temperature or for 5 days in a climatic chamber (heating-cooling cycles, -10°C, +50°C, 10 cycles, 12 h each, wherein 6 h at -10°C and 6 h at +50°C in each cycle).

Both the addition of amorphous diatomaceous earth and the addition of waxes affect the mechanical properties of the produced composites, causing decrease of parameters relative to pure PLA, which is related to the increased brittleness of the materials and is caused by the very characteristics of diatomaceous earth.

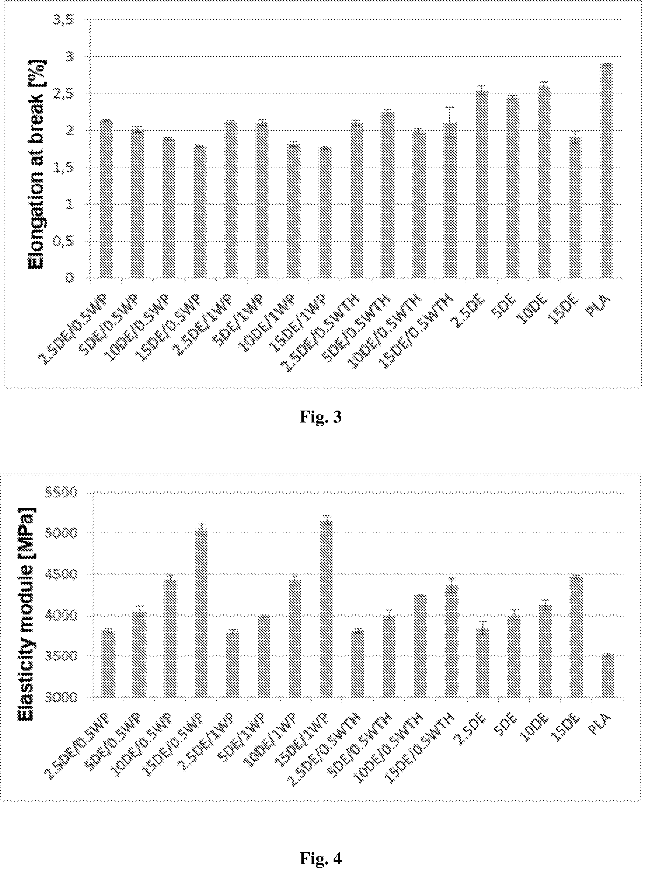

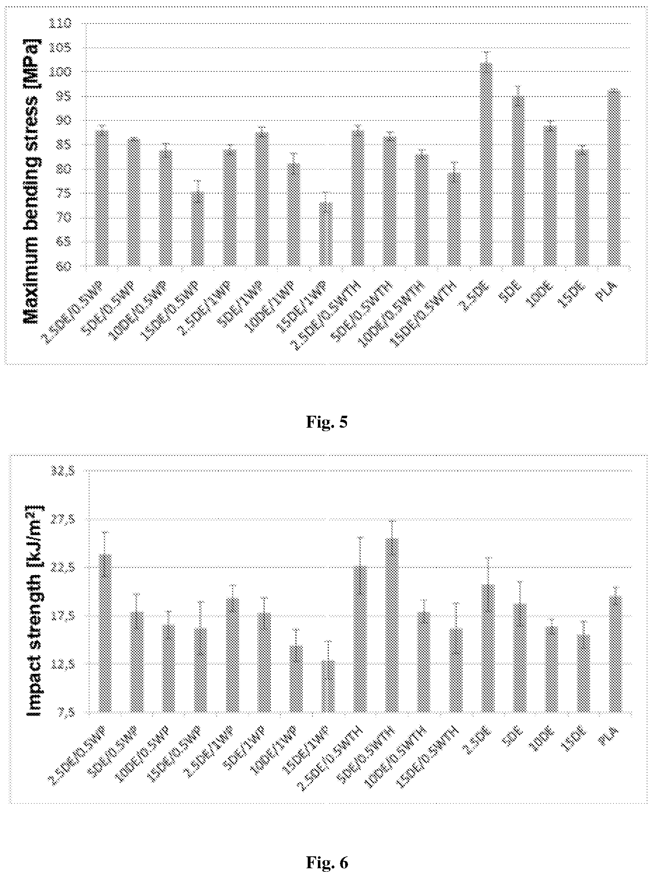

The produced PLA/DE composites were characterized by higher values of elongation at break and thus a lower brittleness than the systems modified with waxes (Tab. 2). With the 2.5% content of diatomaceous earth elongation at break for the systems without the addition of wax was 2.549%, for the systems with the addition of synthetic wax - 2.105% whereas for the systems with 0.5% and 1% addition of natural wax - 2.138% and 2.114%, respectively. As the concentration of DE in the system increased, the strength of the samples decreased (Fig. 2). However, the decrease in the material strength of the samples modified with beeswax was significantly smaller than for the synthetic wax. In the case of beeswax additive, the maximum decrease in the tensile strength value, which was observed in the series depending on the concentration, was 2.45 MPa in the system with 0.5% content of beeswax and for the content of 1% - 3.06 MPa, whereas for the synthetic wax these values amount to maximum 10.28 MPa. The bending strength module for the non-modified PLA was 3.5 GPa (Tab. 3). Modification of PLA with diatomaceous earth in each case, irrespective of the concentration, caused an increase in the value of the parameter. The addition of synthetic wax 0.5% WTH-B did not cause significant changes in the mechanical parameters, irrespective of the concentration, whereas the addition of natural wax noticeably increased the values of bending module. The highest value, 5.16 GPa, was shown by the system with the highest concentration of DE (15%) and 1% addition of beeswax (Fig. 3). Compared with the modification with synthetic wax, an increase by as much as 800 MPa was observed for the systems with the highest concentration of DE. The impact strength for pure PLA was 19.54 kJ/m² (Tab. 4). The addition (2.5%) of diatomaceous earth initially caused an increase in the impact strength (20.76 kJ/m²). With the increase in the concentration of diatomaceous earth, which resulted from an increase in the brittleness of the material, the impact strength decreased. A similar trend was observed in the composites with the addition of natural wax (Fig. 4). The highest values of impact strength for the highest concentration of diatomaceous earth (15%) were obtained in the samples modified with 0.5% content of beeswax - the impact strength was at the level of 16.21 kJ/m², whereas with the same filling for the systems modified with synthetic wax, a slightly lower value was obtained - 16.18 kJ/m², and for pure diatomaceous earth without waxes - 15.53 kJ/m². Conditioning of the composites in the climatic chamber for the period of 5 days caused insignificant differences in the values of elongation at break and tensile strength for pure PLA. The systems modified with diatomaceous earth showed an improvement for the elongation parameter. The highest values were observed for the system with 1% addition of natural wax, up to the value of 3.25% (an increase by nearly 70%) (Fig. 5). At the same time, the conditioning of the composites in the climatic chamber did not significantly affect the bending strength. All the tested systems had similar values of both bending strength and maximum bending stress (Fig. 6).

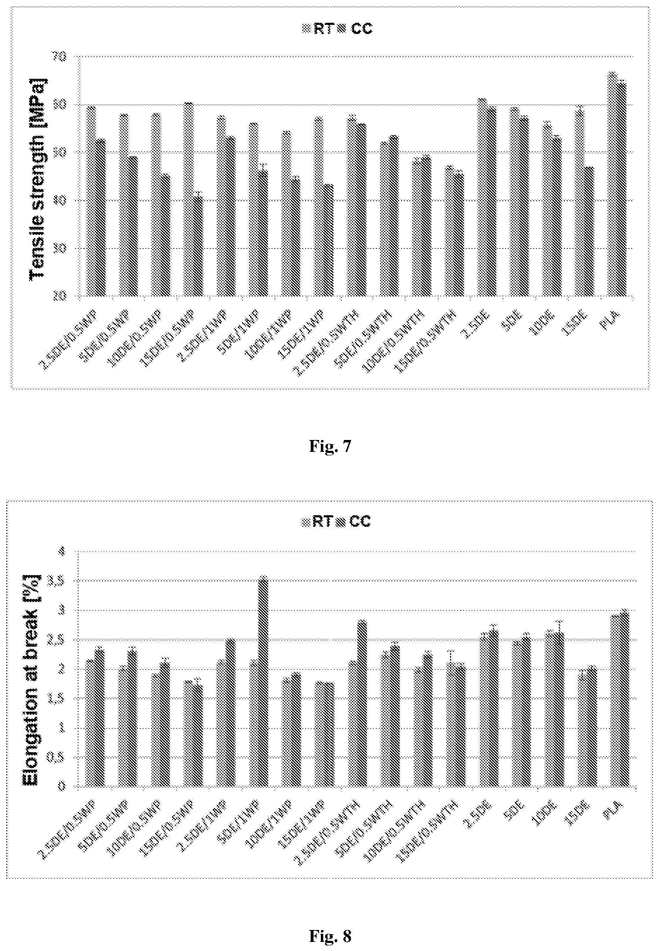

Conditioning in the climatic chamber caused an increase in the impact strength of the composites. The highest results were achieved by the composites with 0.5% addition of natural wax, irrespective of the concentration used, the maximum impact strength was 29.43 kJ/m² (Fig. 7).

The composites modified with diatomaceous earth and beeswax in the majority of cases show an improvement in the mechanical properties, both for the systems conditioned in room temperature and the systems exposed to the influence of adverse atmospheric conditions (elevated temperature, higher humidity). Replacing synthetic wax with natural wax made it possible to not only obtain a full biocomposite but also improve parameters, e.g. processing parameters (a higher MFR) and thus facilitate the process of obtaining the composites.

**Table 2. Tensile strength - systems conditioned at room temperature (RT) and 5 days in a climatic chamber (CC).**

| | **Breaking strength RT [MPa]** | **SD** | **Breaking strength CC [MPa]** | **SD** | **Elongation RT [%]** | **SD** | **Elongation CC [%]** | **SD** |
|---|---|---|---|---|---|---|---|---|
| 2.5DE/0.5WP | 59.38 | 0.28 | 52.556 | 0.639 | 2.138 | 0.014 | 2.327 | 0.091 |
| 5DE/0.5WP | 57.74 | 0.42 | 49.006 | 0.3445 | 2.014 | 0.080 | 2.302 | 0.146 |
| 10DE/0.5WP | 57.85 | 0.31 | 45.098 | 0.817 | 1.887 | 0.031 | 2.109 | 0.145 |
| 15DE/0.5WP | 60.19 | 0.13 | 40.850 | 1.997 | 1.785 | 0.012 | 1.727 | 0.212 |
| 2.5DE/1WP | 57.22 | 0.66 | 53.090 | 0.562 | 2.114 | 0.056 | 2.485 | 0.056 |
| 5DE/1WP | 55.97 | 0.27 | 46.282 | 2.375 | 2.106 | 0.080 | 3.525 | 0.085 |
| 10DE/1WP | 54.16 | 0.52 | 44.406 | 0.991 | 1.808 | 0.063 | 1.905 | 0.066 |
| 15DE/1WP | 56.97 | 0.47 | 43.127 | 0.623 | 1.764 | 0.036 | 1.756 | 0.009 |
| 2.5DE/0.5WTH | 57.21 | 0.98 | 55.886 | 0.130 | 2.105 | 0.062 | 2.788 | 0.061 |
| 5DE/0.5WTH | 51.91 | 0.41 | 53.294 | 0.560 | 2.242 | 0.081 | 2.394 | 0.138 |
| 10DE/0.5WTH | 48.28 | 1.09 | 49.052 | 0.703 | 1.987 | 0.079 | 2.246 | 0.113 |
| 15DE/0.5WTH | 46.93 | 0.58 | 45.480 | 1.4711 | 2.106 | 0.410 | 2.044 | 0.106 |
| 2.5DE | 61.18 | 0.31 | 59.143 | 0.876 | 2.549 | 0.108 | 2.654 | 0.19 |
| 5DE | 59.09 | 0.37 | 57.192 | 0.928 | 2.447 | 0.053 | 2.543 | 0.102 |
| 10DE | 55.78 | 1.28 | 52.987 | 1.120 | 2.607 | 0.100 | 2.612 | 0.387 |
| 15DE | 58.75 | 1.85 | 46.828 | 0.276 | 1.901 | 0.156 | 2.014 | 0.07 |
| PLA | 66.37 | 0.80 | 64.482 | 1.37 | 2.894 | 0.025 | 2.954 | 0.104 |

**Table 3. Bending strength - samples conditioned at room temperature (RT) and 5 days in a climatic chamber (CC).**

| | **Automatic Module RT [MPa]** | **SD** | **Automatic Module CC [MPa]** | **SD** | **Max bending stress RT[MPa]** | **SD** | **Max bending stress CC [MPa]** | **SD** |
|---|---|---|---|---|---|---|---|---|
| 2.5DE/0.5WP | 3808.79 | 52.33 | 3875.16 | 127.68 | 87.93 | 0.979 | 89.78 | 4.77 |
| 5DE/0.5WP | 4049.96 | 117.01 | 4140.63 | 78.67 | 86.21 | 0.392 | 90.01 | 2.337 |
| 10DE/0.5WP | 4442.73 | 97.35 | 4371.96 | 27.14 | 83.84 | 1.447 | 75.84 | 3.462 |
| 15DE/0.5WP | 5055.78 | 149.90 | 4887.23 | 276.66 | 75.44 | 2.259 | 79.10 | 3.413 |
| 2.5DE/1WP | 3801.44 | 46.11 | 3705.34 | 81.70 | 84.03 | 1.061 | 84.57 | 1.544 |
| 5DE/1WP | 3986.93 | 20.46 | 3913.77 | 30.88 | 87.62 | 0.965 | 84.16 | 2.215 |
| 10DE/1WP | 4426.83 | 121.24 | 4192.02 | 196.99 | 81.16 | 2.149 | 79.35 | 3.701 |
| 15DE/1WP | 5156.79 | 100.89 | 4534.11 | 41.84 | 73.21 | 2.101 | 72.31 | 2.641 |
| 2.5DE/0.5WTH | 3808.79 | 52.36 | 3799.215 | 74.85 | 87.93 | 0.979 | 89.69 | 0.729 |
| 5DE/0.5WTH | 4001.11 | 112.42 | 3963.133 | 56.93 | 86.77 | 0.883 | 86.01 | 3.546 |
| 10DE/0.5WTH | 4243.82 | 17.07 | 4175.561 | 125.79 | 83.02 | 0.892 | 85.11 | 4.205 |
| 15DE/0.5WTH | 4363.05 | 169.08 | 4397.266 | 184.36 | 79.32 | 2.042 | 80.54 | 5.178 |
| 2.5DE | 3842.18 | 169.21 | 3851.123 | 122.23 | 101.94 | 2.067 | 100.92 | 1.298 |
| 5DE | 4003.80 | 113.12 | 3998.254 | 193.21 | 95.01 | 2.007 | 96.12 | 2.198 |
| 10DE | 4125.07 | 118.45 | 4118.98 | 98.712 | 88.92 | 1.036 | 90.12 | 1.243 |
| 15DE | 4467.27 | 55.16 | 4369.31 | 95.069 | 83.97 | 0.862 | 77.50 | 3.49 |
| PLA | 3519.54 | 41.37 | 3807.756 | 40.214 | 96.15 | 0.297 | 103.08 | 3.378 |

**Table 4. Impact strength - samples conditioned at room temperature (RT) and 5 days in a climatic chamber (CC).**

| | **Impact strength RT [kJ/m²]** | **SD** | **Impact strength CC [kJ/m²]** | **SD** |
|---|---|---|---|---|
| 2.5DE/0.5WP | 23.86 | 2.27 | 29.43 | 2.32 |
| 5DE/0.5WP | 17.95 | 1.75 | 27.12 | 2.89 |
| 10DE/0.5WP | 16.57 | 1.43 | 20.49 | 2.24 |
| 15DE/0.5WP | 16.21 | 2.71 | 20.84 | 1.00 |
| 2.5DE/1WP | 19.33 | 1.35 | 26.99 | 2.22 |
| 5DE/1WP | 17.78 | 1.61 | 24.65 | 1.88 |
| 10DE/1WP | 14.44 | 1.63 | 22.02 | 0.67 |
| 15DE/1WP | 12.91 | 1.96 | 13.87 | 2.63 |
| 2.5DE/0.5WTH | 22.71 | 2.94 | 23.18 | 3.46 |
| 5DE/0.5WTH | 25.57 | 1.74 | 26.94 | 3.40 |
| 10DE/0.5WTH | 17.93 | 1.18 | 26.20 | 2.64 |
| 15DE/0.5WTH | 16.18 | 2.58 | 25.29 | 4.39 |
| 2.5DE | 20.76 | 2.79 | 21.17 | 2.21 |
| 5DE | 18.76 | 2.30 | 19.47 | 1.98 |
| 10DE | 16.40 | 0.77 | 18.21 | 1.43 |
| 15DE | 15.53 | 1.41 | 19.25 | 2.06 |
| PLA | 19.54 | 0.90 | 24.77 | 1.95 |

### Literature:

1. Aguero A et al.; 2019. Effect of different compatibilizers on environmentally friendly composites from poly(lactic acid) and diatomaceous earth. Polymer Int. 68(5): 893-903
2. Dobrosielska et al.; 2020. Biogenic Composite Filaments Based on Polylactide and Diatomaceous Earth for 3D Printing. Materials; 13(20). 4632.
3. Righetti MC. Cinelli P. Mallegni N. Massa CA. Aliotta L. Lazzeri A. Thermal. Mechanical. Viscoelastic and Morphological Properties of Poly(lactic acid) based Biocomposites with Potato Pulp Powder Treated with Waxes. Materials (Basel). 2019;12(6):990.

## Claims

1. A biocomposite consisting of biodegradable poly(lactic acid) constituting the matrix of the composite, diatomaceous earth constituting the filler and beeswax constituting the rheological factor, wherein the content of individual components is 84-97%, 2.5-15% and 0.5-1%, respectively.

2. A method of producing the composite as defined in claim 1, wherein:
a) poly(lactic acid) in the form of granulate, crude diatomaceous earth constituting 5%, 10%, 20% or 30% of the homogenizate, and beeswax constituting 1% or 2% of the homogenizate, are homogenized in the temperature range from 180°C to 230 °C, preferably 205°C to 215 °C;
b) the components are milled;
c) the product of milling is injected at a temperature of 195°C - 210°C;
d) the product of milling 1:1 is diluted with pure polylactide and a biocomposite with 2.5%, 5%, 10% or 15% content of diatomaceous earth and 0.5% or 1% content of beeswax is obtained;
e) the biocomposite is conditioned for 5 days at room temperature and/or for 5 days in a climatic chamber in 10 heating-cooling cycles (6 h at +50°C and 6 h at -10°C in each cycle).

## Patentansprüche

1. Biokomposit, bestehend aus biologisch abbaubarer Polymilchsäure als eine Matrix des Komposits, Kieselgur als Füllstoff, und Bienenwachs als rheologischem Faktor, wobei der Gehalt der einzelnen Komponenten 84-97 %, 2,5-15 % bzw. 0,5-1 % beträgt.

2. Verfahren zur Herstellung des Komposits nach Anspruch 1, wobei:
a) Polymilchsäure in Form von Granulat, rohe Kieselgur, die 5 %, 10 %, 20 % oder 30 % des Homogenisats ausmacht, und Bienenwachs (1 % oder 2 % des Homogenisats), im Temperaturbereich von 180 °C bis 230 °C, vorzugsweise von 205 °C bis 215 °C, homogenisiert werden;
b) die Komponenten gemahlen werden;
c) das Mahlprodukt bei einer Temperatur von 195 °C bis 210 °C eingespritzt wird.
d) das Mahlprodukt mit reinem Polylactid 1:1 verdünnt wird, wodurch das Biokomposit mit 2,5 %, 5 %, 10 % oder 15 % Kieselgur und 0,5 % oder 1 % Bienenwachs entsteht.
e) das Biokomposit 5 Tage lang bei Raumtemperatur und/oder 5 Tage lang in einer Klimakammer in 10 Heiz-Kühl-Zyklen (6 Stunden bei +50 °C und 6 Stunden bei -10 °C pro Zyklus) konditioniert wird.

## Revendications

1. Biocomposite composé de poly(acide lactique) biodégradable formant la matrice, de terre de diatomées formant la matière de charge et de cire d'abeille formant le facteur rhéologique, dans lequel la teneur de chaque formant est respectivement de 84 à 97 %, 2,5 à 15 % et 0,5 à 1 %.

2. Procédé de production du composite tel que défini à la revendication 1, dans lequel :
a) le poly(acide lactique) sous forme de granulés, la terre de diatomées brute constituant 5 %, 10 %, 20 % ou 30 % de l'homogénéisat, et la cire d'abeille constituant 1 % ou 2 % de l'homogénéisat, sont homogénéisés à une température comprise entre 180 °C et 230 °C, de préférence entre 205 °C et 215 °C ;
b) les composants sont broyés ;
c) le produit de broyage est injecté à une température de 195 °C à 210 °C ;
d) le produit de broyage est dilué à 1:1 avec du polylactide pur, pour obtenir le biocomposite contenant 2,5 %, 5 %, 10 % ou 15 % de terre de diatomées et 0,5 % ou 1 % de cire d'abeille ;
e) le biocomposite est conditionné pendant 5 jours à température ambiante et/ou 5 jours en étuve climatique, avec 10 cycles de chauffage-refroidissement (6 h à +50 °C et 6 h à -10 °C à chaque cycle).
